(19) Europäisches Patentamt / European Patent Office / Office européen des brevets

(11) **EP 4 461 289 A1**

(12) **EUROPEAN PATENT APPLICATION**

(43) Date of publication:
**13.11.2024   Bulletin 2024/46**

(21) Application number: **24171457.5**

(22) Date of filing: **19.04.2024**

(51) International Patent Classification (IPC):
***A61K 8/37*** (2006.01)    ***A61Q 19/00*** (2006.01)

(52) Cooperative Patent Classification (CPC):
**A61K 8/37; A61K 8/375; A61Q 1/02; A61Q 1/06;
A61Q 19/00;** A61K 2800/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   **21.04.2023   TW 112115039**

(71) Applicant: **Patech Fine Chemicals Co., Ltd.
Taipei City 106 (TW)**

(72) Inventors:
• **LIANG, An-Hung
  507 Changhua (TW)**

• **SHIH, Hou-Kuang
  507 Changhua (TW)**
• **PAN, Yu-Zih
  507 Changhua (TW)**
• **LIN, Chia-Ying
  507 Changhua (TW)**
• **HUNG, Jung-Tsung
  507 Changhua (TW)**
• **TSAIH, Jeng-Shiang
  507 Changhua (TW)**

(74) Representative: **Hamer, Christopher K. et al
Mathys & Squire
The Shard
32 London Bridge Street
London SE1 9SG (GB)**

(54)     **COSMETIC ESTER COMPOSITION**

(57)     A cosmetic ester composition includes a trimellitic acid ester which is formed by subjecting a trimellitic acid and a $C_8$-$C_{13}$ alcohol to an esterification reaction, and a $C_5$-$C_{18}$ carboxylic acid polyol ester. The cosmetic ester composition has a hydroxyl value ranging from 5 mg KOH/g to 100 mg KOH/g, and a viscosity at 20°C ranging from 150 cP to 1500 cP.

EP 4 461 289 A1

**Description**

The present disclosure relates to an ester composition, and more particularly to a cosmetic ester composition.

Conventional esters or ester compounds used in the cosmetic field aim to either boost pigment loading capability or improve a user's sensory experience. However, an ester or ester compound that effectively boosts pigment loading capability and improves a user's sensory experience simultaneously has yet to be found.

In view of the aforesaid, there is a need to develop a cosmetic ester composition, which can effectively boost pigment loading capability and improve sensory experience simultaneously.

Therefore, an object of the present disclosure is to provide a cosmetic ester composition, which can alleviate at least one of the drawbacks of the prior art.

According to an aspect of the disclosure, there is provided a cosmetic ester composition according to claim 1.

For the purpose of this specification, it will be clearly understood that the word "comprising" means "including but not limited to", and that the word "comprises" has a corresponding meaning.

It is to be understood that, if any prior art publication is referred to herein, such reference does not constitute an admission that the publication forms a part of the common general knowledge in the art, in Taiwan or any other country.

Unless defined otherwise, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

The present disclosure provides a cosmetic ester composition including a trimellitic acid ester and a $C_5$-$C_{18}$ carboxylic acid polyol ester. The cosmetic ester composition has a hydroxyl value ranging from 5 mg KOH/g to 100 mg KOH/g, and a viscosity at 20°C ranging from 150 cP to 1500 cP. The cosmetic ester composition exhibits excellent pigment loading capability because the hydroxyl value thereof is not lower than 5 mg KOH/g, while ensuring that the hydroxyl value is also not greater than 100 mg KOH/g so as to prevent any sticky and uncomfortable feeling on the user's skin. The cosmetic ester composition exhibits excellent pigment loading capability because the viscosity thereof at 20°C is not lower than 150 cP, while ensuring that the viscosity at 20°C is also not greater than 1500 cP so as to promote excellent spreadability without causing any sticky and uncomfortable feeling on the user's skin. In certain embodiments, the cosmetic ester composition has a viscosity at 20°C ranging from 185 cP to 1450 cP.

According to the present disclosure, the trimellitic acid ester is formed by subjecting a trimellitic acid and a $C_8$-$C_{13}$ alcohol to an esterification reaction. In certain embodiments, the trimellitic acid ester is formed by subjecting the trimellitic acid and a $C_8$-$C_{13}$ saturated monohydric alcohol to an esterification reaction. An example of the trimellitic acid ester may include, but is not limited to, tridecyl trimellitate.

According to the present disclosure, the $C_5$-$C_{18}$ carboxylic acid polyol ester may be selected from the group consisting of a dipentaerythritol ester, a pentaerythritol ester, a glyceride, a polyglycerol fatty acid ester, and combinations thereof.

According to the present disclosure, the dipentaerythritol ester is formed by subjecting a $C_5$-$C_{18}$ carboxylic acid and a dipentaerythritol to an esterification reaction. In certain embodiments, the dipentaerythritol ester is formed by subjecting a $C_5$-$C_{18}$ monocarboxylic acid and a dipentaerythritol to an esterification reaction. Examples of the $C_5$-$C_{18}$ monocarboxylic acid may include, but are not limited to, a $C_5$-$C_{18}$ saturated monocarboxylic acid or a $C_5$-$C_{18}$ unsaturated monocarboxylic acid. An example of the dipentaerythritol ester may include, but is not limited to, dipentaerythrityl pentaisononanoate.

According to the present disclosure, the pentaerythritol ester is formed by subjecting a $C_5$-$C_{18}$ carboxylic acid and a pentaerythritol to an esterification reaction. In certain embodiments, the pentaerythritol ester is formed by subjecting a $C_5$-$C_{18}$ monocarboxylic acid and a pentaerythritol to an esterification reaction. Examples of the $C_5$-$C_{18}$ monocarboxylic acid may include, but are not limited to, a $C_5$-$C_{18}$ saturated monocarboxylic acid or a $C_5$-$C_{18}$ unsaturated monocarboxylic acid. An example of the pentaerythritol ester may include, but is not limited to, pentaerythrityl tetracaprylate/tetracaprate.

According to the present disclosure, the glyceride is formed by subjecting a $C_5$-$C_{18}$ carboxylic acid and a glycerol to an esterification reaction. In certain embodiments, the glyceride is formed by subjecting a $C_5$-$C_{18}$ monocarboxylic acid and a glycerol to an esterification reaction. Examples of the $C_5$-$C_{18}$ monocarboxylic acid may include, but are not limited to, a $C_5$-$C_{18}$ saturated monocarboxylic acid or a $C_5$-$C_{18}$ unsaturated monocarboxylic acid. An example of the glyceride may include, but is not limited to, glyceryl dioleate.

According to the present disclosure, the polyglycerol fatty acid ester is formed by subjecting a C5-C18 carboxylic acid and a polyglycerol to an esterification reaction. In certain embodiments, the polyglycerol fatty acid ester is formed by subjecting a $C_5$-$C_{18}$ monocarboxylic acid and a polyglycerol to an esterification reaction. Examples of the $C_5$-$C_{18}$ monocarboxylic acid may include, but are not limited to, a $C_5$-$C_{18}$ saturated monocarboxylic acid or a $C_5$-$C_{18}$ unsaturated monocarboxylic acid. An example of the polyglycerol fatty acid ester may include, but is not limited to, polyglyceryl-2 diisostearate.

In certain embodiments, the cosmetic ester composition includes the trimellitic acid ester, the dipentaerythritol ester, and the pentaerythritol ester.

In certain embodiments, the cosmetic ester composition includes the trimellitic acid ester and the polyglycerol fatty acid ester.

In certain embodiments, the cosmetic ester composition includes the trimellitic acid ester, the pentaerythritol ester, and the polyglycerol fatty acid ester.

In certain embodiments, the cosmetic ester composition includes the trimellitic acid ester, the dipentaerythritol ester, and the glyceride.

According to the present disclosure, amounts of the trimellitic acid ester and the $C_5$-$C_{18}$ carboxylic acid polyol ester are not particularly limited, as long as the cosmetic ester composition has a hydroxyl value ranging from 5 mg KOH/g to 100 mg KOH/g and a viscosity at 20°C ranging from 150 cP to 1500 cP. In certain embodiments, the trimellitic acid ester is present in an amount ranging from 1 wt% to 99 wt%, and the $C_5$-$C_{18}$ carboxylic acid polyol ester is present in an amount ranging from 1 wt% to 99 wt%, based on the total weight of the cosmetic ester composition.

By virtue of the trimellitic acid ester, the $C_5$-$C_{18}$ carboxylic acid polyol ester, and the hydroxyl value and the viscosity in a specific numerical range, the cosmetic ester composition of the present disclosure can exhibit excellent pigment loading capability and improvement in sensory experience.

The disclosure will be further described by way of the following examples. However, it should be understood that the following examples are solely intended for the purpose of illustration and should not be construed as limiting the disclosure in practice.

## EXAMPLES

### Examples 1 to 4 (EX1 to EX4)

The materials and the amounts thereof for making the cosmetic ester composition of EX1 are shown in Table 1 below. The cosmetic ester composition of EX1 was prepared by uniformly mixing 35 wt% of tridecyl trimellitate with 35 wt% of dipentaerythrityl pentaisononanoate and 30 wt% of pentaerythrityl tetracaprylate/tetracaprate.

In addition, each of the cosmetic ester compositions of EX2 to EX4 was prepared using the corresponding recipe shown in Table 1, and the preparation procedures of these cosmetic ester compositions were similar to those of EX1. A respective one of the cosmetic ester compositions of EX1 to EX4 had a hydroxyl value and a viscosity at 20°C as shown in Table 1. The cosmetic ester compositions of EX1 to EX4 respectively served as test samples EX1 to EX4.

### Comparative Examples 1 to 4 (CE1 to CE4)

Dipentaerythrityl pentaisononanoate served as a test sample of CE1, tridecyl trimellitate served as a test sample of CE2, pentaerythrityl tetracaprylate/tetracaprate served as a test sample of CE3, and polyglyceryl-2 diisostearate served as a test sample of CE4. The hydroxyl value and the viscosity at 20°C of the respective one of the cosmetic esters of CE1 to CE4 are shown in Table 2 below.

Table 1

| Compositional content (wt%) | | | EX1 | EX2 | EX3 | EX4 |
|---|---|---|---|---|---|---|
| | Trimellitic acid ester | Tridecyl trimellitate (CAS number: 94109-09-8) | 35 | 97 | 10 | 2 |
| | Dipentaerythritol ester | Dipentaerythrityl pentaisononanoate (CAS number: 84418-63-3) | 35 | 0 | 0 | 30 |
| | Pentaerythritol ester | Pentaerythrityl tetracaprylate/ Tetracaprate (CAS number: 68441-68-9) | 30 | 0 | 22 | 0 |
| | Glyceride | Glyceryl dioleate | 0 | 0 | 0 | 68 |
| | Polyglycerol fatty acid ester | Polyglyceryl-2 diisostearate | 0 | 3 | 68 | 0 |
| Hydroxyl value (mg KOH/g) | | | 17.1 | 5.0 | 98.9 | 95.0 |
| Viscosity at 20°C (cP) | | | 480 | 1450 | 499 | 185 |

3

Table 2

| | | | CE1 | CE2 | CE3 | CE4 |
|---|---|---|---|---|---|---|
| Compositional content (wt%) | Trimellitic acid ester | Tridecyl trimellitate (CAS number: 94109-09-8) | 0 | 100 | 0 | 0 |
| | Dipentaerythritol ester | Dipentaerythrityl pentaisononanoate (CAS number: 84418-63-3) | 100 | 0 | 0 | 0 |
| | Pentaerythritol ester | Pentaerythrityl tetracaprylate/ Tetracaprate (CAS number: 68441-68-9) | 0 | 0 | 100 | 0 |
| | Glyceride | Glyceryl dioleate | 0 | 0 | 0 | 0 |
| | Polyglycerol fatty acid ester | Polyglyceryl-2 diisostearate | 0 | 0 | 0 | 100 |
| Hydroxyl value (mg KOH/g) | | | 47 | 1 | 1 | 145 |
| Viscosity at 20°C (cP) | | | 3100 | 1546 | 67 | 1336.1 |

Property evaluation

A. Evaluation of pigment loading capability

5 g of the test sample of each of EX1 to EX4 and CE1 to CE4 was uniformly stirred with 3.5 g of a pigment (i.e., iron oxide yellow or D&C Red No. 7 Calcium Lake), so as to obtain a color paste. Subsequently, the color paste was gradually added with 0.5 g of the pigment at a time, and its glossiness and ease of stirring was observed until the addition of the pigment caused a surface of the color paste to lose its glossiness or difficulty in stirring occurred (i.e., the last addition of the pigment). The pigment loading of the test sample of each of EX1 to EX4 and CE1 to CE4 was determined by calculating the total amount of the pigment, including the 3.5 g of pigment initially added plus the total amount of the pigments added from the first addition to the penultimate addition, and the pigment loading rate (%) of the test sample of each of EX1 to EX4 and CE1 to CE4 for evaluating the pigment loading capability was calculated using the following Equation (1):

$$A = (B/C) \times 100\% \qquad\qquad (1)$$

where

A= pigment loading rate (%)
B= pigment loading (i.e., 3.5 g of pigment initially added plus total amount of pigments added from the first addition to the penultimate addition)
C= amount of each test sample (i.e., 5 g)

B. Sensory evaluation

The test sample of each of EX1 to EX4 and CE1 to CE4 having a size of approximately a green bean was applied by the evaluators to the skin on the inner side of their arms. Subsequently, the test sample of each of EX1 to EX4 and CE1 to CE4 were subjected to evaluation of spreadability, play time, cushion feeling, smoothness, and non-stickiness feeling according to the following scoring criteria.
Spreadability: referred to as the ease with which the test sample could be pushed away by the evaluators, and was scored out of 10 (first decimal place acceptable). A higher score indicates that the test sample has better spreadability and thus are more easily pushed away.
Play time: during the application of the test sample on the evaluators' skin, the evaluators rated the time period taken for the skin to perceive a sensation generated by the test sample, which was scored out of 10 (first decimal place acceptable). A higher score indicates that the test sample remains tactile to the evaluators' skin for a longer period of time, and thus can be applied on the skin for a longer period of time.

Cushion feeling: during the application of the test sample on the evaluators' skin, the evaluators rated the degree to which the test sample provided a thick and resilient sensation on the skin, which was scored out of 10 (first decimal place acceptable). A higher score indicates that the test sample provides the evaluators with a more pronounced thick and resilient sensation on their skin.

Smoothness: the evaluators rated the degree to which the test sample provided smoothness on their skin, which was scored out of 10 (first decimal place acceptable). A higher score indicates that the test sample provides the evaluators with a smoother sensation on their skin.

Non-stickiness feeling: the evaluators rated the degree to which the test sample provided non-stickiness feeling on their skin, which was scored out of 10 (first decimal place acceptable). A higher score indicates that the test sample provides the evaluators with a less sticky sensation on their skin.

The results of the pigment loading capability and sensory evaluation for the test sample of each of EX1 to EX4 and CE1 to CE4 are summarized in Table 3 and Table 4.

Table 3

| | | | EX1 | EX2 | EX3 | EX4 |
|---|---|---|---|---|---|---|
| Evaluation of pigment loading capability | Pigment loading rate (%) | Iron oxide yellow | 44.4 | 44.4 | 50 | 54.5 |
| | | D&C Red NO. 7 Calcium Lake | 60 | 60 | 61.5 | 61.5 |
| Sensory evaluation | Rating score | Spreadability | 4.4 | 4.3 | 4.5 | 5.0 |
| | | Play time | 4.8 | 5.3 | 4.5 | 5.5 |
| | | Cushion feeling | 5.1 | 5.5 | 5.3 | 5.0 |
| | | Smoothness | 4.8 | 4.5 | 4.8 | 5.3 |
| | | Non-stickiness feeling | 5.2 | 4.0 | 5.0 | 5.2 |

Table 4

| | | | CE1 | CE2 | CE3 | CE4 |
|---|---|---|---|---|---|---|
| Evaluation of pigment loading capability | Pigment loading rate (%) | Iron oxide yellow | 41.6 | 38.5 | 43.4 | 54.1 |
| | | D&C Red No. 7 Calcium Lake | 51.3 | 50 | 55.3 | 56.5 |
| Sensory evaluation | Rating score | Spreadability | 2.3 | 3.2 | 6.5 | 3.7 |
| | | Play time | 3.0 | 3.5 | 5.3 | 4.5 |
| | | Cushion feeling | 6.3 | 4.3 | 2.5 | 5.3 |
| | | Smoothness | 2.3 | 3.7 | 5.2 | 3.8 |
| | | Non-stickiness feeling | 2.3 | 3.5 | 6.7 | 4.0 |

Referring to Table 3 and Table 4, compared with the cosmetic esters of CE1 to CE4, the cosmetic ester compositions of EX1 to EX4 exhibited higher pigment loading rate, better spreadability, longer play time, more pronounced cushion feeling, better smoothness, and enhanced non-stickiness feeling.

Summarizing the above test results, it is clear that the cosmetic ester composition of the present disclosure, including the trimellitic acid ester and the $C_5$-$C_{18}$ carboxylic acid polyol ester, with the hydroxyl value ranging from 5 mg KOH/g to 100 mg KOH/g and the viscosity at 20°C ranging from 150 cP to 1500 cP, simultaneously exhibits excellent pigment loading capability and provides the user's skin with comfortable attributes (i.e., a pronounced cushion feeling, a high degree of smoothness, a high spreadability, an extended play time, and a non-sticky sensation).

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

**Claims**

1. A cosmetic ester composition, **characterized by**:

   a trimellitic acid ester which is formed by subjecting a trimellitic acid and a $C_8$-$C_{13}$ alcohol to an esterification reaction; and
   a $C_5$-$C_{18}$ carboxylic acid polyol ester;
   the cosmetic ester composition having a hydroxyl value ranging from 5 mg KOH/g to 100 mg KOH/g, and a viscosity at 20°C ranging from 150 cP to 1500 cP.

2. The cosmetic ester composition as claimed in claim 1, wherein the trimellitic acid ester is formed by subjecting the trimellitic acid and a $C_8$-$C_{13}$ saturated monohydric alcohol to an esterification reaction.

3. The cosmetic ester composition as claimed in any one of claims 1 and 2, wherein the $C_5$-$C_{18}$ carboxylic acid polyol ester is selected from the group consisting of a dipentaerythritol ester, a pentaerythritol ester, a glyceride, a polyglycerol fatty acid ester, and combinations thereof, the dipentaerythritol ester being formed by subjecting a $C_5$-$C_{18}$ carboxylic acid and a dipentaerythritol to an esterification reaction, the pentaerythritol ester being formed by subjecting a $C_5$-$C_{18}$ carboxylic acid and a pentaerythritol to an esterification reaction, the glyceride being formed by subjecting a $C_5$-$C_{18}$ carboxylic acid and a glycerol to an esterification reaction, the polyglycerol fatty acid ester being formed by subjecting a $C_5$-$C_{18}$ carboxylic acid and a polyglycerol to an esterification reaction.

4. The cosmetic ester composition as claimed in claim 3, wherein the dipentaerythritol ester is formed by subjecting a $C_5$-$C_{18}$ monocarboxylic acid and a dipentaerythritol to an esterification reaction.

5. The cosmetic ester composition as claimed in claim 3, wherein the pentaerythritol ester is formed by subjecting a $C_5$-$C_{18}$ monocarboxylic acid and a pentaerythritol to an esterification reaction.

6. The cosmetic ester composition as claimed in claim 3, wherein the glyceride is formed by subjecting a $C_5$-$C_{18}$ monocarboxylic acid and a glycerol to an esterification reaction.

7. The cosmetic ester composition as claimed in claim 3, wherein the polyglycerol fatty acid ester is formed by subjecting a $C_5$-$C_{18}$ monocarboxylic acid and a polyglycerol to an esterification reaction.

8. The cosmetic ester composition as claimed in any one of claims 1 to 7, wherein the trimellitic acid ester is present in an amount ranging from 1 wt% to 99 wt%, based on the total weight of the cosmetic ester composition.

9. The cosmetic ester composition as claimed in any one of claims 1 to 8, wherein the $C_5$-$C_{18}$ carboxylic acid polyol ester is present in an amount ranging from 1 wt% to 99 wt%, based on the total weight of the cosmetic ester composition.

Europäisches Patentamt

European Patent Office

Office européen des brevets

# EUROPEAN SEARCH REPORT

**Application Number**

EP 24 17 1457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HÖLLERER CHRISTINE ET AL: "Comprehensive monitoring of specific metabolites of tri-(2-ethylhexyl) trimellitate (TEHTM) in urine by column-switching liquid chromatography-tandem mass spectrometry", ANALYTICAL AND BIOANALYTICAL CHEMISTRY, SPRINGER BERLIN HEIDELBERG, BERLIN/HEIDELBERG, vol. 410, no. 18, 24 April 2018 (2018-04-24), pages 4343-4357, XP036535476, ISSN: 1618-2642, DOI: 10.1007/S00216-018-1086-7 [retrieved on 2018-04-24] * page 4344; compounds 5OH-1-MEHTM, 5OH-2-MEHTM * | 1 | INV. A61K8/37 A61Q19/00 |
| | ----- | | |
| X | DATABASE GNPD [Online] MINTEL; 4 October 2018 (2018-10-04), anonymous: "Cover Me Pro Artist Liquid Concealer", XP093205919, Database accession no. 6021837 | 1-3,6-9 | |
| Y | * the whole document * | 1-9 | TECHNICAL FIELDS SEARCHED (IPC) |
| | ----- | | A61Q A61K |
| X | DATABASE GNPD [Online] MINTEL; 5 March 2013 (2013-03-05), anonymous: "Brilliant Plump Lip Gloss", XP093205758, Database accession no. 2011849 | 1-5,8,9 | |
| Y | * the whole document * | 1-9 | |
| | ----- | | |
| Y | US 2009/214456 A1 (GREENBERG STEPHEN [US] ET AL) 27 August 2009 (2009-08-27) * paragraphs [0002], [0037]; example 1 * | 1-9 | |
| | ----- | | |
| | -/-- | | |

The present search report has been drawn up for all claims

| Place of search | Date of completion of the search | Examiner |
|---|---|---|
| Munich | 23 September 2024 | Skulj, Primoz |

EPO FORM 1503 03.82 (P04C01)

page 1 of 2

Europäisches
Patentamt

European
Patent Office

Office européen
des brevets

**EUROPEAN SEARCH REPORT**

Application Number

EP 24 17 1457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | "NONCHEMICAL SUNBLOCK", SOAP COSMETICS CHEMICAL SPECIALTIES, MAC.NAIR-DORLAND CO. NEW YORK, US, vol. 68, no. 9, 1 September 1992 (1992-09-01), pages 107-108, XP000350514, ISSN: 0091-1372 | 1-3,6,8,9 | |
| Y | * "Nonchemical sunblock" * | 1-9 | |
| | ----- | | |
| X | WO 2023/054898 A1 (COSMAX INC [KR]) 6 April 2023 (2023-04-06) | 1-4,6,8,9 | |
| Y | * claim 11; examples 1-5; table 5 * | 1-9 | |
| | ----- | | |

**TECHNICAL FIELDS SEARCHED (IPC)**

The present search report has been drawn up for all claims

| Place of search | Date of completion of the search | Examiner |
|---|---|---|
| Munich | 23 September 2024 | Skulj, Primoz |

EPO FORM 1503 03.82 (P04C01)

## ANNEX TO THE EUROPEAN SEARCH REPORT
## ON EUROPEAN PATENT APPLICATION NO.

EP 24 17 1457

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2024

| Patent document cited in search report | | Publication date | Patent family member(s) | | Publication date |
|---|---|---|---|---|---|
| US 2009214456 | A1 | 27-08-2009 | AR | 057349 A1 | 28-11-2007 |
| | | | AT | E527023 T1 | 15-10-2011 |
| | | | BR | PI0611012 A2 | 22-02-2011 |
| | | | CN | 101188992 A | 28-05-2008 |
| | | | EP | 1888011 A1 | 20-02-2008 |
| | | | JP | 2008542498 A | 27-11-2008 |
| | | | KR | 20080028885 A | 02-04-2008 |
| | | | US | 2009214456 A1 | 27-08-2009 |
| | | | WO | 2007011449 A1 | 25-01-2007 |
| WO 2023054898 | A1 | 06-04-2023 | KR | 20230045413 A | 04-04-2023 |
| | | | WO | 2023054898 A1 | 06-04-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 94109-09-8
- *CHEMICAL ABSTRACTS,* 84418-63-3
- *CHEMICAL ABSTRACTS,* 68441-68-9